# EUROPEAN PATENT APPLICATION

(11) **EP 4 389 038 A1**
(43) Date of publication of application: **26.06.2024**
(21) Application number: 23213761.2
(22) Date of filing: 01.12.2023
(51) Int. Cl.: A61B 18/12, A61B 18/00

(54) **ELECTRO SURGICAL GENERATOR FOR FEEDING AND CONTROLLING ELECTRO SURGICAL INSTRUMENTS**

(30) Priority: 23.12.2022 US 202263435012 P; 23.12.2022 US 202263435003 P
(71) Applicant: Olympus Winter & Ibe GmbH, 22045 Hamburg (DE)
(72) Inventor: Krüger, Jens, 15738 Zeuthen (DE); Stopp, Fabian, 10318 Berlin (DE); Janich, Fabian, 14469 Potsdam (DE); Kwik, Anne, 14165 Berlin (DE)
(74) Representative: Eisenführ Speiser

(57) **Abstract**

Electro surgical generator for generating feed signals for feeding electro surgical instruments, and the generator comprises a plurality of functional modules and the plurality of functional modules comprises at least one inverter module for generating a feed signal for providing a high frequency energy for at least one electro surgical instrument, and at least one output module for connecting an electro surgical instrument to the output module, wherein at least one, a plurality or all of the functional modules comprises a coupling identifier for making the respective functional module distinguishable from other of the functional modules which are different to the respective module, at least one of the functional modules or a communication module of the electro surgical generator are adapted to identify the respective functional module by the coupling identifier, and a bus system is provided for communication between the functional modules and/or with the communication module, and/or each of a plurality or all of the functional modules have a control base module for controlling the respective functional module and/or for operating a communication of the respective functional module with another functional module and/or with the communication module .

## Description

The present invention is directed to an electro surgical generator for generating feed signals for feeding electro surgical instruments. The present invention is also directed to an assembly of at least two of such electro surgical generators. The invention is also directed to a method for manufacturing and operating at least one of such electrical surgical generators.

Electro surgical generators, which could also be called electro surgical generator platforms, are used to operate electro surgical instruments. At least one electro surgical instrument in operation is connected to such electro surgical generator. Such electro surgical instrument can be a device for cutting tissue of a human body, or treat the tissue in a different way that uses electric energy, such as burning it. There are different kinds of electro surgical instruments which can be supplied with high frequency currents of different frequencies, depending on the device. An electro surgical instrument can also be supplied with an electrical signal having an ultra-sonic frequency.

An electro surgical generator having one or more electro surgical instruments connected can be seen as an electro surgical system. Hospitals and other medical practices may need different electro surgical systems, i.e. having one or a plurality of electro surgical instruments and the electro surgical instruments being of different type, depending on the needs of said medical practice.

Accordingly the electro surgical generator is adapted to the electro surgical instruments which are connected or which are available to be connected according to the particular needs of the medical practice.

For example a simple electro surgical system may just have one single electro surgical instrument for just one kind of operation or similar operations according to the kind of medical treatments in said medical practice. Such electro surgical instrument just needs one socket for being connected to the electro surgical generator. Also only one possibly small inverter is needed in order to provide the high frequency feed signal to the electro surgical instrument.

Accordingly the electro surgical generator is built to match this needs. However in other cases more and/or different electro surgical instruments are needed. Such electro surgical instruments can be connected to the electro surgical generator at the same time or one after another. Different electro surgical instruments may need different sockets. Such different socket can be adapted to the mechanical counter socket at the electro surgical instrument and it can also be adapted to the amount and/or kind of power needed by the electro surgical instrument.

Accordingly different sockets may be needed and different inverters possibly as well. The result may also be that more elements in the electro surgical generator are needed when compared to the above-mentioned simple solution.

Accordingly to fulfil all these needs an electro surgical generator is individually designed for the particular electro surgical system. Such design includes the kind and number of inverters, the kind of number of socket, the kind and number of displays, the kind and number of input devices such as nobs or touch displays, to give just a few examples.

Accordingly the problem arises that many different electro surgical generators need to be planned and supplied. For designing, planning and manufacturing such large number of possible variations the risk of manufactural errors is increased. In addition all modules built in such electro surgical generator need to be adapted to each other to ensure that they are functioning correctly.

As electro surgical systems are used for treatments at the human body, there is also a big risk involved of hurting the patient. Accordingly there is also a high level of safety requirements for such electro surgical systems. One safety requirement can be, that the interaction of modules must be ensured and in particular functioning such as quickly stopping the supply of energy to an electro surgical instrument must also be ensured. Such proper functioning depends on a safe communication and/or interaction of modules.

For example an electro surgical instrument may send an error message if a sudden need arises to stop the energy supply to this electro surgical instrument. Such signal thus will be sent to an inverter or to a control base module controlling the inverter and the signal must be understood as being such emergency signal. Understanding emergency signal may depend on what kind of electro surgical instrument is connected and what kind of signal the inverter or controller of the inverter is expecting from this electro surgical instrument. If there is a mismatch of any of these modules, then such emergency signal might be understood incorrectly.

Accordingly if such individual electro surgical generator is build, there might be a risk that even the correct modules might be built into this electro surgical generator but maybe one of the modules is incorrectly identified and thus the communication is not working well. This could lead to misunderstanding said emergency signal, to revert to the above mentioned example.

Accordingly the object of the present invention is to suggest a solution to at least one of the above mentioned problems. In particular a solution shall be provided that enables the manufacturing of an electro surgical generator in many different configurations in a safe and reliable way. At least a solution shall be suggested which is an alternative to so far known solutions.

According to the invention an electro surgical generator according to claim 1 is suggested. In the following the electro surgical generator may also synonymously be denominated generator.

Such generator is designed for generating feed signals for feeding electro surgical instruments. In particular the electro surgical generator may generate an ac-voltage having a particular frequency, in particular having a high frequency in the region of 300 - 4000 kHz. The feed signal may also be in the region of ultrasonic, in particular in the range of 20kHz to 100kHz, in particular approx. 50 kHz.

The generator comprises a plurality of functional modules and the plurality of functional modules comprises at least the following modules.

The plurality of functional modules may comprise an input power module for supplying the electro surgical generator with electrical power. Such input power module may in particular be a power supply, also known as mains unit. This input power module is basically transforming the voltage of an electrical supply grid down to a voltage level that can be used by the generator. One input power module can be enough and can be provided in different sizes depending of the size of the generator. However it is also possible to have more than one input power module. In particular if different voltages are needed by modules that shall be connected to such input power module.

The plurality of functional modules also comprises at least one inverter module for generating at least one voltage signal to be used as a feed signal for at least one electro surgical instrument. The inverter module is coupled to the input power module. Accordingly the input power module provides a low voltage that can be used by the connected inverter module. If there are more than one inverter modules, these inverter modules can at least partially be connected to the same input power module, or one inverter module can be connected to one input power module and another inverter module can be coupled to another input power module, such that the output module of the input power module matches the input voltage of the inverter module.

Based on that the inverter module can produces an ac-voltage having a particular frequency, i.e. a high frequency or a ultrasonic frequency, depending on the electro surgical instrument that shall be supplied by the corresponding inverter module.

The plurality of functional modules also comprises at least one output module for connecting an electro surgical instrument to the output module.

Such output module can in particular be a socket module or it can be denominated as socket module. Such output module is designed to receive a counter socket from the electro surgical instrument that shall be connected.

Accordingly the socket of such output module or socket module matches the counter socket of the corresponding electro surgical instrument. That may include to provide an electrical connection between the output module and the electro surgical instrument in order to supply an electrical feed signal from or via the output module to the electro surgical instrument. In addition there might be an information connection that enables to submit information between the output module and the connected electro surgical instrument. That may also be just a pin or similar sensor identifying whether the electro surgical instrument is plugged in or not. Also information of whether the user has pulled a handle of the electro surgical instrument could be submitted.

In the simplest form there is just one output module but there may be a plurality of output modules for connecting different electro surgical instruments. At least one of the output modules may be provided for having an ultrasonic instrument connected.

The plurality of functional modules may comprise also an energy distribution module for controlling the feed of power from at least one of the inverter modules to the at least one of the output modules. In particular each inverter module may be connected via the energy distribution module to the output module. This way a feed signal may be provided by the inverter module, being transferred over the energy distribution module to the output module and thereby to the connected electro surgical instrument. By conducting the feed signal generated by the inverter module through the energy distribution module to the output module the energy distribution module can control this feed signal. However the inverter module, or at least one inverter module, could also be directly connected to an output module and the feed signal generated by said inverter module can be controlled by the energy distribution module by controlling the inverter module. In particular the energy distribution module may control the inverter module by sending reference signals and by sending start and stop signals for starting or stopping the generating of a feed signal.

The electro surgical generator may further comprise a communication module, which could also be denominated as communication unit, coupled with two or more of the functional modules for providing a communication between the communication module and the two or more functional modules or in-between the two or more functional modules. Such communication module can thus be used to control any of the functional modules to forward informations from one functional module to another, or to identify functional modules and/or operating states of functional modules including whether an electro surgical instrument is plugged into an output module.

Such communication can be build up in a centralized or decentralized way. If it is build up in a centralized way, than the communication module is accordingly a central communication module receiving and sending most or all data signals. This way all the communication goes via the communication module. It is also possible to use a decentralized communication network according to which functional modules directly communicate with each other. The communication module may nevertheless be used to execute some central operations such as identifying an overall state of the generator and/or electro surgical system.

It is further suggested that at least one of the functional modules comprises a coupling identifier for making the respective functional module distinguishable from other of the functional modules which are different to the respective functional module. It is further suggested that the communication module and/or at least one of the functional modules are adapted to identify the respective functional module by the coupling identifier.

Accordingly a functional module or a plurality of the functional modules and in particular all functional modules can be identified, at least they are different from the respective functional module. In otherwords identical functional modules may have identical coupling identifier, i.e. identical functional modules have an identical identification. Different functional modules have a different coupling identifier and thus a different identification, i.e. a different ID.

Identifying of the functional modules based on the coupling identifier can be done by the communication module, i.e. by a central unit, which the communication module will then be. This way the communication module can check which functional modules are used in the generator. However in a decentralized structure this identification can be done by other functional modules. In particular the other functional modules can identify each other.

It is to be noted that the described electro surgical generator is already manufactured or is at least in the process of being manufactured. Accordingly all modules including the communication functional are fixed in the generator. Nevertheless providing said coupling identifier, in particular such that all functional modules have this coupling identifier, enables the generator to check if the correct functional modules are implemented in the generator.

It also enables the correct connection and incorporation of the functional modules into the generator.

If there is a structure of the complete generator given, in particular deposited in the communication module or otherwise in the generator, the interconnection of all functional modules can be done based on that. A functional module can be identified by its coupling identifier and based on that the adequate communication protocol can be implemented.

By the coupling identifier the system knows which functional module is connected and it knows which communication protocol to use and thereby each communication signal received by said functional module is understand correctly. On the other hand it is also clear which communication signal is to be sent to the particular identified functional module.

In addition other characteristics of each functional module are known once it is identified by the coupling identifier. For example a particular functional module be it in an inverter module or an output module having an electro surgical instrument connected may have a limit value such as a current or power limit. If such limit is exceeded the corresponding inverter can be controlled to send a feed signal below this limit or to shut down if such high energy signal is an indication of a malfunction. Such reaction on exceeding a limit can result if the inverter exceeds one of its limits or the inverter sends a feed signal which exceeds the limit of a connected output module with a corresponding electro surgical instrument connected.

This way it is possible to manufacture the generator by just mounting the modules, also attaching the communication module physically in particular to a casing or to a support frame such as a conductor board. In addition the functional modules and the communication module can be connected at least for the exchange of information using the bus system. Accordingly the functional modules and the communication module can be connected to such bus system. That can according to one embodiment be done automatically after physically connecting the modules and the communication module to said conductor board or similar board.

In addition at least for the functional modules that provide, submit or consume power, these can be connected electrically in a way that electrical power can be submitted.

Afterwards just by identifying the coupling identifiers all functional modules can be identified and in a first step it can be checked if the correct functional modules are connected. It can be checked if any functional module is missing, if there is a functional module that should not have been provided and/or if there is a functional module which has obviously and inadvertently been provided instead of another functional module. In this case of course the module that has been installed by mistake can be exchanged.

Assuming that - with or without exchange - all correct functional modules are attached, all communication protocols and safety measures can be implemented for each functional module.

For example if an output module has been identified which shall receive at most a feeding current of 20 Amps this limit is implemented in the system in particular in the communication module and/or in the energy distribution module. Based on that it can always be ensured, that this limit of 20 Amps is not exceeded. If on the other hand the limit was identified to be just 10 Amps, a corresponding limit is implemented.

The use of such coupling identifier is also helpful in case of exchanging a functional module later on. If such module is change later on, the old module ay have different properties than the new module. However by using the coupling identifier it is automatically clear that the module has new properties and the system, in particular the communication module and/or the energy distribution module, can adapt any operation to this new module.

This way also an additional functional module can be provided and embedded into the existing system. Such embedding is not only simple but also avoiding any errors.

The coupling identifier can be provided in different ways. One possibility is to have a software identifier which is read out by the communication module, or other connected functional module. This can particularly be done via the communication bus. Any possible identifiers can be listed in a table which can be deposited, i.e. stored in the generator, in particular in the communication module and by receiving the coupling identifier any necessary information of such functional module identified by its coupling identifier can be read out of such list.

However it could also be possible to have a mechanical identifier. Such mechanical identifier can be used alone or in addition to a software identifier.

However the most favourite embodiment would be that all functional modules have a coupling identifier being provided as a software identifier which can be read out once the module is connected. However the software identifier can also be fixedly implemented in each module, e.g. using a ROM or using an EPROM.

According to one aspect it is suggested that each of a plurality or all of the functional modules have a control base module for controlling the respective functional module and/or for operating a communication of the respective functional module with another functional module and/or with the communication module.

Accordingly the control base module is adapted for controlling the function of the module and/or for controlling the operation of the communication. In particular, for controlling the function of the functional module the base control module may in its most simple form switch it on or off. Also control values and control orders can be put into practice for controlling the function of the functional module.

For communicating the control base module is responsible to send and receive information frames via the communication network in particular via a corresponding bus system. In other words any protocol for communication is implemented in the control base module.

Preferably there is a module detector provided for detecting which functional modules are mounted and/or are configured. Which functional modules are mounted can be identified by analyzing the coupling identifier of each functional module. For detecting the configuration of each functional module the module detector can communicate with the particular functional module.

It is also suggested that a bus system is provided for communication between the functional modules and/or with the communication module. This way, the functional modules may be provided in a modular way. Also, the electrosurgical generator can be manufactured in a modular way but still a communication between the functional modules can be established. That can be directly between the functional modules, or via the communication module.

Preferably the communication module and the functional modules are all installed and/or mounted in or on one support frame and/or casing building the electro surgical generator. Thus the electro surgical generator forms one entity.

According to one aspect it is suggested that two or more of the functional modules each having a communication interface for communicating with other functional modules and/or with the communication module and/or a self-configuring communication network is provided, and in particular at least the communication module and at least one of the functional modules are adapted to search for communication members connected to the network and to autonomously connect to any members found by this search. Wherein the members could in particular be functional modules and/or comprise the communication module.

Therefore these functional modules and in particular all functional modules, have a communication interface and communicate with other functional modules and/or with the communication module. These functional modules and/or the communication module can be used to identify at least another functional module. This can be done by searching for other functional modules on the communication network to which all this elements are connected. To do this at least one of these functional modules and/or the communication module may send a request signal for functional modules for the search to be answer. Depending on the communication system, in particular depending on the bus system used it is also possible that all functional modules connected to the communication system automatically start sending a signal, in particular sending an identifier including their coupling identifier. The functional module searching for other functional modules receive such signals and based on the identifier can recognize a functional module that is already known or a functional module that needs to be embedded into the communication system and thus into the generator at all.

This way all functional modules and/or the communication module can search for other functional modules until all functional modules have been found and embedded and this way the communication network is configured and in fact has configured itself.

According to one embodiment the communication network is designed such that each functional module needs to identify itself at the communication module. This way each functional module sends an identifier, in particular the coupling identifier, and the communication module receives this information and connects the functional module into the communication network.

According to one aspect a plurality of the functional modules each have a control base module for controlling the functional module, wherein the functional module is prepared to provide at least one module function, and in the control base module a modular control method is implemented, that has at least a communication control module for operating the communication of the module and a function control module controlling the function of the module. A module function is thus what the function module is provided for. The module function of the inverter is to provide the feed in signal. The function of the energy distribution module is to control the feed out power from at least one inverter module to at least one output module. The module function of a display module is to display information. The module function of an input module is to forward information received from a user.

Each of such functional modules has a control base module and in this control base module a modular control method is implemented. The method is in such a way modular that one part is directed to controlling the operation of the communication and the other part is directed to controlling the function of the module. Accordingly the communication control module is responsible to send and receive information frames via the communication network in particular via a corresponding bus system. The function control module for controlling the function of the module is directed to the particular functioning of the module.

E.g. the function control module of the inverter may control semiconductor switches inside the inverter in order to provide a high frequency signal. The function control module of the energy distribution module controls switches in orderto direct a feed signal from the correct inverter module to the correct output module.

Therefore according to this aspect at least a plurality of functional modules has an additional communication control module taking over the communication with the communication module and/or other functional modules, namely with the communication control modules of other functional modules. Such communication control modules can be identical for different functional modules. For example, the communication control module of an inverter module can be the same as the one for an output module and/or for another inverter module, to give just one example. There must be a difference in the coupling identifier, which may be implemented in the communication control module, but the functioning as such of the communication control module may be identical only that different identifiers will be send.

This way a communication structure can be provided which can be programmed and controlled in a basically homogenous way whereas the functional modules regarding their module function can be quite different. That also facilitates the manufacturing of the generator in a modular way as all modules may be provided with the same communication control module, only an individual coupling identifier must be implemented.

That the functional modules each have a control base module for controlling the functional module provides a modular structure in the electro surgical generator, as all functional modules may basically be controlled autonomously. If a functional module is exchanged, its control base module is thus exchanged as well. No new design of the overall generator is necessary but all remaining modules can be left unchanged.

According to one aspect in each of at least one of the plurality of functional modules a plurality of function control modules is provided, each as a software module for controlling different module functions of a functional module such that each module function is assigned to a function control module and for selecting a module function a corresponding function control module is selected.

In particular an inverter module may provide different feed signals and thus different module functions. Such different feed signals may be different in view of frequency and/or in view of duration and/or in view of current limits. In particular depending on an application and in particular depending on an electro surgical instrument the inverter module may function differently adapted to the situation in particular adapted to the connected electro surgical instrument.

Accordingly such inverter module can be flexible in its application and this way it is also possible to not provide too many different inverter modules but to only provide this different software control units. Accordingly different operational programs are implemented and are there to choose from.

Another example is a display module displaying the status of possible functions including possible electro surgical instruments to be used. Such display may have a particular given size and on this display there can be shown the status of all possibly connected electro surgical instruments. To make a simple example if three such instruments can be connected, there can be one status symbol for each of these three instruments. However in another electro surgical generator there might be only two instruments and accordingly only two status symbols are needed.

Continuing this example the physical display and thus the functional module can be the same in both cases. Only the function control module that is used is different. Based on the given example there is at least one function control module prepared to show three status symbols and another function control module is prepared to show two status symbols. Of course there can be more than these two function control modules, as each is just provided as a software module. So the physically identical display module can be used for said different generators. Such display modules are mounted in the generator and embedded in the communication system. Based on the coupling identifier the system, in particular the communication module identifies the particular display module. That may include that by knowing the identifier the physical size of the display is known but also the possible function control modules to choose from are also known. Thus it is known that one function control module provides three status symbols whereas the other function control module provides only two status symbols.

In addition it is identified how many output modules are installed in the generator. One possibility is that two output modules are installed and identified and each output modules is only capable for one particular instrument to be connected. Accordingly it is identified that a display module with only two status symbols is needed. Therefore based on that the corresponding functional control unit of the display module is selected.

Accordingly only few display modules need to be provided, maybe even only one display module is needed. However the generators may vary in size such that also different display modules with different physical sizes might be needed. However only few display modules are needed and it is thus avoided to place a wrong one as there are only a few possibilities to choose the wrong one and in addition these few display modules are different in their physical size and thus it will be quite obvious if the wrong display module will be tried to be attached, as it would not fit to the space provided for the display module. However in addition to facilitating the manufacturing of the generator the use of different functioning control modules also provides a high flexibility in case of changes to the generator or generator system.

For example if later on a further output module shall be installed as a further electro surgical instrument shall be added, the display can stay the same. It is only necessary, to add said third output module. This third output module can be identified when being connected to the communication network, in particular the communication bus system and will thus be embedded in the generator. The communication module, or other module in the generator, will than identify that there is a third instrument connected and can then select from the function control modules of the display module that function control module that provides a module function having three status symbols for connected instruments.

A further example can be the output module which can also synonymously be denominated as socket module. Such socket module can include a supervisory function in particular a function that checks the amplitude of a current provided through this output module to a connected instrument. Depending on the instrument to be connected such amplitude may be different. Such functioning may be provided and thus implemented in the different function control modules. So if different instruments are connected and the output modules need to be adapted to these different instruments this can be done by selecting the adequate function control module.

According to one aspect for controlling each functional module a plurality of control base modules are provided as software units and for varying a functionality of the functional module at least one of the control base modules is changeable and/or it can be chosen between some of the control base modules wherein in particular at least one of the control base modules is provided as fixed unchangeable control base module.

This aspect is similar to the one explained above. However according to this aspect the main aim is to be able to make changes of the functionality. Such changes are not necessarily related to exchanging or adding instruments or of changing the configuration of the generator in any other way. Instead the possibility is given to provide a changed functionality with the same elements, also the same instruments according to a corresponding demand. This demand can be given by a user of the generator system.

This way such changes can be performed such that a control base module can be changed.

For example it might be implemented that a feed signal, in particular a feed current shall not jump from 0 to a particular amplitude but may raise according to a temporal ramp function. This temporal ramp function may have a particular slope and this particular slope can be provided by the control function. This can be done by the control function of the inverter or the control function of the output module, or both.

For changing this a variable of the actual control base module which is implemented as software, can be changed. For example the slope can be changed by 10 % to be more or less steep, to give just an example.

Of course this change can also be done by changing between preconfigured control base modules, but it might be preferred to just change a variable if anything else of the control base module and thus anything else of the functionality of said module remains unchanged.

According to one option it is suggested that at least one of the control base modules, i.e. per functional module is provided as fixed, unchangeable control base module. The idea is that this unchangeable control base module can also be a reliable basis. Based on this reliable bases changes to other control base modules can be made. According to one example there is a further control base module which is identical to the fixed, unchangeable control base module, but this second control base module can be changed. This way a varied control base module can be achieved based on the unchangeable control base module and if necessary any changes can be taken back to the original configuration of the control base module as given by the unchangeable control base module. In anyway a higher flexibility of the generator system can be given and it is possible to implement updates in this way in a simple and efficient manner.

According to one aspect a configuration file is given including predetermined configurations in order to select and/or set control base modules, which are particularly given as software units. Optionally the electro surgical generator, in particular the communication module, is adapted to conduct a check, whether the actual chosen and/or set control base module matches the predetermined configuration.

Accordingly the electro surgical generator can be manufactured by using the physically needed functional modules. These functional modules may each change in size and may be according to its general function, such as if an inverter is for providing ultrasonic feed signals or other feed signals, but other than that only few variations of the modules are possible. In other words there are only few different modules each. However such few modules may provide many more variations in functioning. However when manufacturing a particular electro surgical generator there is a particular configuration on what such generator is intended to be. So after physically connecting all modules, including the communication control module, the modules can be adapted according to this predetermined configuration which is provided by the configuration file.

For example the configuration file and thus the configuration may include information on which inverter shall be connected to which output module. It may also include what kind of functionality the inverter modules and/or the output modules shall have. Further functionalities can be predetermined in said configuration, such as what the display of the display module shall look like when in operation.

Based on this configuration file and after the functional modules have been interconnected by the communication network, in particular functionalities are selected or set according to the configuration file.

Similar as explained above a control base module, which can be given as a software unit, can be given together with many other control base modules for the same functional module. So for configuring the generator system it can be chosen from such a plurality of control base modules. However instead or in addition such control base modules i.e. particular parameters of such control base modules can be set to values given by the configuration file.

This way many configurations are possible even though only very few different elements, i.e. only very few different functional modules are needed.

As an additional aspect it is possible to use such configuration file to check if the actually given configuration of the generator matches this given configuration file. This way it can be checked whether incorrect choices and/or settings have been taken and in case of any differences this can be corrected. However it is also possible to check the configuration according to inherent functionality that may not be changed of some or all functional modules. In other words this way it can also be checked whether incorrect functional modules have been installed. This avoids errors when manufacturing the generator. Accordingly this option can be used for checking the physically configuration of the generator just after all modules have been attached and connected to the communication network, i.e. the bus system. So in an early stage such errors can be recognized and fixed.

According to one aspect it is suggested that the electro surgical generator, in particular its communication module is adapted to execute one, a plurality or all of the communication steps of the list comprising:
- receive information from functional modules,
- evaluate the information received,
- extract control commands from the evaluated information,
- transmit control commands extracted from the evaluated information,
- transmit received information to other functional modules and
- check if transmitted information, in particular transmitted control commands, matches the functional module to which they were transmitted.

First of all it is important to mention that in an electro surgical generator it is not necessary that all modules communicate. In known generators it is also possible that there is no communication structure at all.

However the present invention is particularly directed to providing an electro surgical generator which can be manufactured in an easy efficient and particularly modular way. Using such modular manufacturing of the electro surgical generator can be improved or may be only made possible using a good communication in between a functional module and the communication module. This way any adaption of functional modules can be done by this communication. Any simple information, such as switching a functional module on or off or at least starting or ending a standby modus could be done without a communication system in particular without a bus system. But here it is suggested to use such communication to also implement simple information transmittal such as a start or stop but also if an instrument is connected or not to give further example.

The communication suggested is thus so complex that it provides a possibility to execute one, a plurality or all of the mentioned communication steps.

One step is receiving information from functional modules. That implies that functional modules do sent information.

Even further such information received is evaluated. This also implies that the information is complex enough that it can be evaluated. In particular information send and/or received or evaluated can include an information frame which is frequently send and indicates identification, information and functional information which is actually used to control a functional module.

Control commands can also be send and such control commands can be received by evaluating such information that has been received before. This way the communication system can be used to provide corresponding control commands.

Such control commands can be transmitted further. For example an output module can transmit information to the communication module. The communication module can receive such information evaluated and identify the send control command. Such control command could for example may be to let the inverter stop generating the feed signal, or let the inverter start generating the feed signal or let the inverter change the generated feed signal. Accordingly such command received by the communication module can then be forwarded to the particular inverter. The information received from the output module, to stick to this example, could include which inverter shall start, stop or change its feed signal.

However it is also possible that one of the communication steps is just to transmit received information to other functional modules. This can particularly be a step done by the communication module and in particular if the data structure is centralized.

The communication system also enables the possibility to check if transmitted information matches the functional module to which they were transmitted. This way any errors can be avoided or at least the number of errors can be reduced. If transmitted information, in particular transmitted control commands, do not match the functional module to which there were transmitted, the configuration can be double checked and possibly the functional module can be changed and/or the functional module which sent the control command can get a new configuration such that it then sends the correct control commands.

According to one aspect the communication module is coupled to the functional modules via a communication wire. In addition or alternatively the communication module is connected to a bus system for communication, in particular to a serial bus system. Accordingly the communication module and thus the connected functional modules use a bus system for communication. It was realized, that the electro surgical generator includes at least one inverter module and such inverter module produces high frequency feed signals and thus also sends electric smog of various frequencies which may disturb any communication systems which is inside the same casing as these inverter. Accordingly a communication wire can shield any communication transmitted via such communication wire against such electric smog. However a complex communication in between the communication device and the functional modules can be provided by a bus system. It was found that a serial and/or differential bus system is better protected against said interfering signals and thus it is suggested to use a serial and/or differential bus for the communication.

According to one aspect it is suggested that the electro surgical generator, in particular the communication module, comprises or uses a plurality of bus systems having different rates of transmission. It was found that different tasks and different modules in the electro surgical generator need differently urgent communication. In particular there are safety sensitive operations and commands that need to be communicated and executed quickly. One of this examples is to stop the feed of a feed signal, in particular stopping a feed current resulting from the feed voltage. The output module, i.e. the socket module can identify a situation according to which the feeding needs to be stopped urgently. This can either be identifying that the feed current exceeds a limit or it can be an error signal received by the attached instrument. It is also possible that according to the behavior of the feed current such urgent situation is identified.

As a result such output module sends a signal to reduce or stop the feed signal to the inverter module. That can be depending on the communication structure via the communication device.

The time needed by the inverter for stopping the feed signal can be in the range of about 100 m/s. Accordingly any communication for stopping such signal should be much quicker at least not longer.

On the other hand a display module does not need to react such quickly. First of all the display module is just providing information to the user which often does not even need any reaction. In addition the user is hardly able to identify such quick change on a display.

One solution could be to provide a fast bus system to all modules as a fast bus system can of course also support any process that does not need a quick communication. However in that case there is a risk of overloading the bus system and/or overloading the communication module which needs to evaluate all the information provided on the bus system. Accordingly it is suggested to have different bus systems with different rates of transmission. That may also include that such different bus systems may use the same data lines but also different data lines can be used for different bus systems. If different data lines are used it is also possible to have an improved level of shielding transmission of wires against high frequency radio signals for safety relevant signals.

The different transmission rates may also be used for different situations. In particular if the electro surgical generator is in a standby mode, no urgent communication is needed and in fact there is no need for stopping any feed signal as there is currently no feed active. It is also possible that only some modules are in standby mode, i.e. one attached electro surgical instrument is in standby mode, whereas another electro surgical instrument is in operation. Then the high transmission rate can be used for the instrument that is in operation whereas the slower transmission rate can be used for the instrument that is in standby mode.

According to one aspect the electro surgical generator, in particular the communication module, is adapted to select a transmission rate for exchanging information, depending on information to be transmitted and/or depending on the module to be controlled.

This selection of this transmission rate is also based on the explanations given above. I.e. depending on the information to be transmitted and thus depending on the task to be handled, in particular the task to be controlled, the transmission rate shall be selected. Initiating any safety relevant actions or any actions that are potentially safety relevant, is potentially more urgent.

Accordingly corresponding information is send with a high transmission rate. Other information can be send with a lower transmission rate. It was also understood that some modules are in general not safety relevant and/or according to other reasons not involved in urgent action. Accordingly for such modules it can in general be selected to use a lower transmission rate.

On the other hand there are modules which are generally safety relevant and thus it is suggested to generally use a high transmission rate for such safety relevant modules. It was also found that providing different transmission rates and in particular providing the possibility to select different transmission rates facilitates the modular concept of the suggested electro surgical generator. This suggestion provides the possibility to have a communication system, in particular the communication module and the bus system that is capable of transmitting different kind of information and thus capable of controlling different kinds of modules.

Accordingly such communication system can be provided in a general manner and can be adapted in particular modules connected to the communication system, thus connected to the communication device. The particular way of communication in particular the transmission rate to be selected does not need to be preprogrammed adapted to the particular electro surgical generator to be manufactured. Instead it can just be provided as a general component. As only a high transmission rate is provided for some modules and/or some situations it is also possible to provide enough capacity for different configurations of the electro surgical generator. This also enhances the modular concept.

According to one aspect the electro surgical generator, in particular the communication module is adapted to identify a connection situation reflecting an overall situation of functional modules currently installed and/or currently connected in the electro surgical generator. In particular that is done depending on the identified connection situation and overall control function of the electro surgical generator for controlling the electro surgical generator.

The electro surgical generator may have different functional modules installed and/or currently connected. In principle an operation of the generator all functional modules are installed and connected. However it is also possible to only identify the installed, in particular mounted modules it is also possible to just identify connected modules. Both can lead to the same result but it is possible, that modules are not connected completely and on the other hand modules connected do not necessarily be mounted.

However these situations may be identified by the generator in particular the communication module and this way the actual configuration of the electro surgical generator is identified. It is thus possible, to install and connect all modules that are planned for the electro surgical generator. Based on that the electro surgical generator, in particular the communication device can identify the overall situation themselves. That can in particular be done by using the coupling identifier. According to these coupling identifiers it is known which of the modules are there, including their kind and theirtype. I.e. Including what kind of module is there, i.e. if it is an inverter module, output module or display module to just give some examples. The type can on the other hand specify for example the size of the particular module, such as how much power can be generated, forwarded and/or otherwise used.

The result is thus the overall situation and this enables the generator to select an overall control function of the electro surgical generator. Such overall control function may include how the functional modules interact. It can include, to give an example, which inverter shall prove a feed signal to which output module. It may include how display modules are incorporated into the control system and how input modules are also incorporated. For example the control function can control whether to receive input signals from a display module or not, depending on whether the display module is also capable of receiving input signals of a user, in particular if that display module has a touch screen which enables such input.

The output modules may be adapted for particular electro surgical instruments. There are at least three basic different electro surgical instruments available which need particular feed signals and particular connection and also observation.

One instrument is a bipolar electro surgical instrument that receives as a feed signal an ac-current - simply speaking - flowing into the instrument for being used for operational treatment on the tissue of the body to be operated and flowing back to the output module.

Another kind of instrument is a monopolar instrument that receives an ac-current as a feed signal which flows into the instrument and into the body where it is also used for some kind of treatment but this instrument uses an external electrical ground. Accordingly the feed signal is basically further flowing through the body of the patient to a ground to which the patient is connected.

Another general kind of instrument is an ultrasonic instrument that receives an ac-feed signal having a ultrasonic frequency, in particular having a value of about 50 kHz which makes the operating heads of the instrument vibrate with said ultrasonic frequency. Such vibration might result in heating this operational head for using this heat for the operation task. This instrument may also be connected in a bipolar way. Accordingly, the way to control these instruments can be preprogrammed and selected according to the identified connection situation. In particular depending on the size of output modules it may also be possible to adapt the control function.

The control of the particular feed signal may be one characteristic of the overall control function. However there are much more interactions to be considered by such overall control function. For example if a display displays a feed current on a scale of 0 % to 100 %, this 100 % must be adapted to the maximum value of the corresponding feed signal. Accordingly depending on the module used this maximum value of the feed signal may be different and the overall control function can be adapted to such particulars.

A further part of the overall control function might be which kind of safety measures to be taken. Such safety measures may depend on the size of the electro surgical instruments that may be connected according to the size of the output module.

Accordingly it can be ensured that the correct overall control function is used if it is automatically selected and/or adapted depending on the connection situation. That avoids that an incorrect control function is installed. It also enables a change of modules in the electro surgical generator which can simply be done by exchanging the particular modules. Having a new module than leads to a different connection situation and this will be identified and the control function is adapted and/or selected accordingly.

According to one aspect for communication, the communication module is interconnected with the functional modules via communication lines, and/or the functional modules are interconnected with each other via communication lines and/or a bus system is used, in particular a bus system using a serial and/or differential data transfer, in particular a CAN-bus.

Accordingly, there are communication lines for communication between the functional modules. This communication can be provided directly or via the communication module. Depending on this topology, the communication lines are provided accordingly.

This communication can be done by a bus system and thus the communication lines are part of the bus system. Preferably, a bus system using a serial and/or differential data transfer, such as a CAN-bus, is used. It was found that due to the inverter modules a high interference resistance i.e. an immunity against electro smog is advisable and therefore it is suggested using a serial and/or differential data transfer. In particular, a CAN-bus uses such serial data transfer and can use a differential transfer structure as well. The CAN-bus is thus helpful to increase the interference resistance against noise coming from the inverter module.

According to one aspect, the electro surgical generator, in particular the communication module uses a plurality of bus systems, having different transfer rates. This use of a plurality of bus systems using different transfer rates increases safety features of the electro surgical generator. It must be kept in mind that the electro surgical generator provides electrical current flowing in and possibly through the body of a patient. Bus systems may lead to problems with electrical isolation. Using at least two separate bus systems using different transfer rates improves the safety in view of this electrical isolation.

It was thus found that the use of the electro surgical instrument may lead to an electrical circuit in which the patient forms part of this electrical circuit. The bus system should be isolated to such electrical circuit. In addition using separate bus systems, the risk of loosing an isolation in between different electrical circuits is reduced.

The different transfer rates may also improve the noise immunity of the communication within the electro surgical generator and thus between the communication module and/or the functional modules.

According to one aspect, a plurality or all of the functional modules each comprise a data interface for building up a communication or for participating in a communication with the communication module and/or with other functional modules. Accordingly, many, most or all functional modules are provided with such communication interface. This communication interface may be part of a control base module of the corresponding functional module and in particular it is part of a communication control module of such control base module and thus of such functional module. This way, a modular design of the electro surgical generator can be achieved. Each functional module is having such data interface which can also be denominated as communication interface and can thus basically be used for any electro surgical generator and can be connected with different functional modules and/or with the communication module.

In order to do that, the data interface is even adapted to build up a communication with another module. This ability can be given by building or preparing the data interface such that it initially sends data frames and/or receives and analyzes data frames available on a communication system such as a bus system to which the data interface can be connected.

Accordingly, such functional module having such data interface only needs to physically be connected with the communication module and/or other functional modules. After that, a communication can be built up, in particular, it can be built up automatically.

It is also possible that a communication network already exists and in that case the data interface enables the functional module to participate in such communication network.

According to one aspect, at least one of the functional modules comprises settable or selectable module configurations. Accordingly, the functional module is not completely fixed in its configuration. Such configuration may comprise how to interact with other functional modules or the communication module. Such configuration can comprise parameters to be set, such as a limit of an output value or a frequency value of a feed signal. One possibility is that a configuration module exists but particular parameters are set and in this case the module configuration is settable. Another possibility is that there are different module configurations prepared for each functional module to choose from. In this case, there is a selectable module configuration.

In particular, the electro surgical generator is adapted that for each of the functional modules a module configuration is set or selected in dependence of an identified setup of the electro surgical generator. Such identified setup may either be what kind of functional modules are provided in the particular electro surgical generator. Another possibility is that the setup of the electro surgical generator is given by a setup file. Reading out such setup file can also be identifying the setup.

Accordingly, the configuration of the functional module can be set or selected in order to comply with the setup of the electro surgical generator. An electro surgical generator may be provided for a particular use such as what kind of electro surgical instruments shall be used and thus connected. The setup of the electro surgical generator may depend on the particular field of operation which the customer is ordering the electro surgical generator is specialized in. Based on that, different feed signals may be provided by the inverter modules, including different amplitudes and/or different frequencies. It is also possible to interconnect the electro surgical generator with external data files or equipment. There may also be different provisions regarding safety issues which may depend on the particular use of the electro surgical generator but these may also depend on governmental requirements. All this can be part of the setup and based on that, the module configuration of the functional modules is set or selected.

According to one suggestion, each functional module having settable or selectable module configurations comprises a configuration unit. Such configuration unit is thus comprising the settable or selectable module configuration and the setting or selecting can be done using this configuration unit. The configuration unit may be connected to the communication module and receive orders to set or select the module configuration. However, the configuration unit may as well comprise some functionality such as being able to check whether such orders for setting or selecting the module configuration are adequate and/or possible.

In addition or alternatively, a central configuration unit is provided, in particular, as part of the communication module. This way, the configuration of all module configurations can be done in a centralized way. This also facilitates the setting or selecting of the module configurations depending on the setup of the electro surgical generator.

The setup of the electro surgical generator can be understood as what kind of functional modules are installed in the electro surgical generator. Accordingly, the setup can also be denominated as module setup as it identifies which kind of modules are installed in the electro surgical generator.

According to one aspect, an enabling function is implemented wherein the enabling function is adapted to enable a particular functional module to operate and/or to execute a particular function, such that the particular functional module can only operate or execute the particular function if it is enabled by the enabling function, wherein for enabling a central enabling unit is provided and/or decentralized enabling units are provided such that each functional module comprises an enabling unit.

This way, a high safety standard can be reached. This also enables the modular design of the electro surgical generator. All modules can be mounted in the electro surgical generator and connected for communication. They can further possibly be configured and controlled by means of a communication within the generator. In particular, such communication can be conducted by the communication module and this way the functional modules can also be prepared for use.

However, in order to avoid any malfunction and in order to avoid any dangerous generation of feed signals that could harm a patient, there is in addition this enabling function. The enabling function may, e.g. based on a configuration file, checks if the particular module is configured correctly, operating correctly and/or interconnected correctly. If that is the case, the functional module can be enabled to operate.

It is also possible that a particular function of the functional module is enabled. In particular, it is suggested that the communication provided by the functional module is not affected by the enabling function, i.e. that the communication of the functional module always works and is thus always allowed. However, the particular function that shall be provided by the particular functional module should be enabled, e.g. if the functional module is an inverter module generating a feed signal shall be enabled by the enabling function. If the functional module is a distribution module forwarding such feed signal, this forwarding of the feed signal shall be enabled. If the functional module is an output module, i.e. a socket module, such output module shall at least be enabled to also forward a feed signal to a connected electro surgical instrument.

The enabling function may check whether the particular functional modules are without error. However, in addition or alternatively, the enabling function may also or only check whether a particular path for a feed signal is complete such that all involved functional modules are in place and connected. E.g., such path may be a path for a feed signal from an inverter module possibly via a distribution module to an output module. If all these modules are in place, correctly connected and not having any error, the enabling function may enable all functional modules of this path.

Enabling can be done by sending an enabling flag to the particular functional modules. It is also possible that such enabling function is implemented in each functional module and the functional module is enabling itself or enabling its own function. The information needed for this enabling can be received via a communication network between the particular functional modules, in particular a bus-system, possibly conducted by the communication module.

According to one aspect, the electro surgical generator comprises a configuration file, having stored an intended setup of the electro surgical generator, defining which functional modules to be used in the electro surgical generator, and/or the electro surgical generator being adapted to compare, in particular by means of a comparator, the setup of the electro surgical generator stored in the configuration file with an actually identified setup of the electro surgical generator.

This also facilitates the modular concept of the electro surgical generator, in particular manufacturing such electro surgical generator. The manufacturing is done based on a predetermined configuration. This predetermined configuration is also stored in the configuration file. Once the electro surgical generator is assembled, i.e. all functional modules and possibly further modules are mounted. The resulting and thus actual setup of the electro surgical generator can be double-checked using this configuration file. This way, it is avoided that an incorrect electro surgical generator is produced.

One way of checking whether the actual setup is correct, a comparator can be used comparing the intended setup as stored in the configuration file with an actually identified setup of the electro surgical generator. The identification of the actual setup of the electro surgical generator can be done by using the coupling identifier of each functional module or other component.

According to one aspect, the electro surgical generator comprises further modules as follows.

A further functional module may be a display control module having a control algorithm for controlling a display module. In particular, the display control module is adapted to identify a display module installed in the electro surgical generator and adapt the control algorithm to the identified display module. Accordingly, it is suggested having a display module as a functional module and as a further separate module said display control module. This is also to facilitate the modular character of the electro surgical generator, as there is suggested to have one display control module for different display modules. In each electro surgical generator, one of these various display modules is installed and it is controlled by the display control module. The display control module may be the same in another electro surgical generators each having a different display module.

Therefore, only one display control module, or only a few of these, need to be designed and need to be available when manufacturing an electro surgical generator. Once both modules are installed in the electro surgical generator, the display control module may identify which display module is installed and adapt its control accordingly.

Another possible functional module of the electro surgical generator may be an external communication module having a communication protocol for communicating with external applications connected to the external communication module. Accordingly, this external communication module is part of the electro surgical generator but adapted for communicating with external applications. Such external applications could be an apparatus for a secretion suction. This can for example be stopped and started depending on the operation of the electro surgical instrument operated by the electro surgical generator. An automation of the medical operation may also be an external application. This could include controlling equipment or lights in the operational room. Another external application could be an electronic file of the patient. A further external application could be a cloud based connection for updates or for additions of functions of the electro surgical generator.

In particular, it is suggested that the external communication module is adapted to identify connected external applications, and to adapt the communication protocol to the identified external applications. This way, the external communication module could be the same for different electro surgical generators and the external communication module can then just apply itself to the particular external applications identified.

Another functional module of the electro surgical generator may be an input module for enabling a user to give inputs to the electro surgical generator. It receives any input from a user such as pressing a knob or identifying an amplitude of a feed signal or inputting a time for a duration for a particular function. All these input signals can be received by such input module and forwarded for further processing into the electro surgical generator, in particular transferred to the communication module.

A further module of the electro surgical generator may be a module detector for detecting functional modules installed in the electro surgical generator. Such module detector can be used to detect and thus identify what kind of functional modules are installed. Optionally, it can also detect any other modules installed. This way, it is possible to identify the setup of the electro surgical generator and possibly adapt the electro surgical generator accordingly as explained above.

According to one aspect, the electro surgical generator is adapted to receive and process an upgrade signal. Preferably, the electro surgical generator comprises an upgrade processing unit to receive and process an upgrade signal. Such upgrade signal identifies new or amended functionality. For example, if it was found that for a particular operation process of a patient a particular feed signal turned out to be better than what was so far used, such new feed signal can be part of such upgrade signal. The upgrade signal thus identifies such changed feed signal, i.e. with respect to amplitude and/or frequency, and also provides further information needed to be implemented. Such information could include limits that need to be adapted as such changed feed signal may not only affect the inverter module but also the corresponding output module and possibly a distribution module. The change of frequency and amplitude was just a simple example and the change may be more complicated and such more complicated change may also need an adaption of how to display such signal and accordingly the upgrade signal may also include information on how to adapt the display module and/or a display control module. All this information can be part of the upgrade signal.

The electro surgical generator may be adapted to receive and process such an upgrade signal by having a particular software implemented, in particular implemented in the communication module. However, it is also possible and it is suggested as an option to provide an upgrade processing unit for receiving and processing an upgrade signal, as a separate module. Such upgrade processing unit may comprise an own communication interface for physically receiving the upgrade signal. Such interface can be an interface socket for connecting a data line. The interface socket or interface module could for example be a USB-port, to give a particular example.

Receiving and processing an upgrade signal can be directed to a software update of the system, i.e. a software update of the electro surgical generator, in particular implemented on the communication module, or it could be a software update of particular functional modules.

The upgrade could also be for an extension of the electro surgical generator, such as implementing additional applications which could apply additional procedures. These could be handling procedures or also particular operational procedures.

Limits of feed signals, in particular power limits, could be changed as part of an upgrade. It is also possible that already prepared functions, i.e. preprogrammed in the electro surgical generator such as in the communication module, will be firstly enabled by an upgrade signal.

Also as part of an upgrade, additional modes could be downloaded and installed, such as modes directed to particular provisions of countries, including permission of particular modes. It is also possible to permit and/or enable special modes which are rarely used but now shall be used. Such rarely used modes could usually be disabled in order to reduce the complexity of the system.

Existing modes could also be updated by the upgrade signal. Such update could be adapting a particular feed signal that was already there, i.e. specified by length, amplitude and frequency but is now slightly adapted regarding at least one of these values. This way, an optimization could be achieved.

An upgrade could also be directed to integrating further external applications, in particular equipment. Such external applications or equipment could be an automation of the operation room where the operation at the patient takes place. It could also be the control of a secretion suction and/or a data transfer to an electronic patient file.

According to one aspect, the electro surgical generator comprises a support frame or casing on which all functional modules and in particular also all other modules are mounted, and in particular the support frame or casing comprises a power supply for supplying electrical power to the electro surgical generator, in particular to all modules that need electrical power.

Accordingly, the electro surgical generator is one single device basically housed in a casing. However, some modules are according to their nature attached at the casing, in particular at the outside of the casing, in order to facilitate interaction to electro surgical instruments being connected, to a user and/or to other external applications. Accordingly, the modular concept of the electro surgical generator is providing an apparatus as a single piece and the electro surgical generator is not spatially distributed and/or separated in separate housings spaced apart.

Instead, all the functioning and in particular the communication in between all modules takes place on such support frame or in such casing. Of course, the support frame and the casing may be combined and the support frame may also or alternatively be comprised by two or more printed boards. Such printed boards can be mounted inside such casing. However, the electro surgical generator might as well go without casing, maybe only with a front plate, if it is prepared for being inserted into a control cabinet, to give just one example.

It is however just important that all modules are mounted in such a way that they form one physical unity.

The invention is also directed to a method for manufacturing an electro surgical generator which is adapted for generating feed signals for feeding electro surgical instruments, and the generator comprises a plurality of functional modules and the plurality of functional modules comprises
- at least one inverter module for generating a feed signal for providing a high frequency energy for at least one electro surgical instrument,
- at least one output module for connecting an electro surgical instrument to the output module,
wherein
- at least one, a plurality or all of the functional modules comprises a coupling identifier for making the respective functional module distinguishable from other of the functional modules which are different to the respective module, and
the method comprises the steps
- mounting at least the functional modules to a casing or support frame,
- building up a communication between the functional modules, and
- identifying each functional module by its coupling identifier, and
- using a bus system for communication between the functional modules and/or with the communication module and/or
wherein
- each of a plurality or all of the functional modules have a control base module for controlling the respective functional module and/or for operating a communication of the respective functional module with another functional module and/or with the communication module and
wherein in particular
- an electro surgical generator according to any of the preceding claims is manufactured.

Accordingly the electro surgical generator can be manufactured in an easy and reliable way. The functional modules can be identified by their coupling identifier. The functional modules can be mounted basically in a regular manner to a casing or support. Once these functional modules and in particular once all components of the electro surgical generator that need to be mounted are mounted, a communication is built up between these functional modules. A communication can be built up between further modules as well. However the communication shall be built up between the functional modules at least. This can be in a decentral or central way. Accordingly functional modules can be interconnected for communication directly or a communication can be done via a communication module. Such communication module can be a central module which can control and conduct the communication between the functional modules.

Accordingly data frames can be exchanges directly in between functional modules and/or between the functional modules and the communication module.

So once the modules are mounted and a communication is build up, each functional module can be identified by its coupling identifier. This is thus done via the communication between these functional modules and/or between the functional modules and the communication module. The result of the identification of the functional modules can be stored in the communication module. If there is a decentralized communication structure and there is no communication module, the communication can be stored in one of the functional modules, or in some or all of the functional modules.

Accordingly after mounting and building up the communication all functional modules can be identified. As a result it is known during manufacturing which functional modules have been mounted. This way all functional modules are identified and thus it is identified which functional modules are mounted in the electro surgical generator. Accordingly a configuration of the electro surgical generator is known and accordingly the details of such electro surgical generator are known. In other words the electro surgical generator has been identified and this way it can be double checked whether the correct functional modules have been mounted.

Preferably an electro surgical generator according to any of the aspects explained above for an electro surgical generator is manufactured. Electro surgical generators having different configurations can be build in the same manner. Similar but not identical functional modules can be used and mounted to the casing or support frame. Just depending on the particular functional modules used and mounted, different configurations and thus different electro surgical generators can be manufactured.

Identifying each functional module by its coupling identifier can be performed such that each functional module sends a data frame including such coupling identification, namely an identification number. In addition there may be a database provided listing possible coupling identifier, i.e. listing the corresponding identification number and having assigned characteristics of the corresponding functional module. Such characteristics can include what kind of functional module is assigned to the identification number, i.e. if it is an output module, an inverter module or any functional module or even other module.

The characteristic may further include the size of the functional module and possibly what kind of communication protocol is implemented in the functional module. Depending on the type of module the characteristic may comprise a current limit, if the functional module is an output module having such current limit. The characteristically value could be a number of buttons and what kind of buttons, if the functional module is an input module. The characteristic may comprise a number of display digits, if the functional module is a display module. Accordingly all the characteristics are known, once the functional module is identified. It is also possible that just the identification number of each identified functional module is stored.

Accordingly using this coupling identifier enables a manufacturing in a modular way. Therefore the functional modules just need to be mounted and afterwards the particular overall functioning of the electro surgical generator can be set just based on that, once all mounted functional modules are identified.

According to one aspect based on the identified functional modules a generator composition is identified. Accordingly as described above identifying the functional modules not only provides this particular information on each single module but enables the identification of the electro surgical generator as a whole.

In addition or alternatively the electro surgical generator comprises a communication module for controlling the communication between the functional modules and/or for controlling the building up of the communication. According to this aspect such communication module is suggested as a module having a central function for the communication. Such communication module can be used for controlling the communication between the functional modules, i.e. the communication can be controlled such that a communication between two functional modules is executed via the communication module. Accordingly said functional modules send data frames for communication not directly to the corresponding other functional module. E.g. a first functional module may send a data frame to the communication module and the communication module sends a corresponding data frame to a second functional module and this said first and second functional module communicate in this way indirectly via the communication module. However said first and second functional module may in addition communicate with each other.

The communication module can also be used for building up a communication. This can be done such that once all functional modules are mounted, the communication module starts sending requests to potential functional modules or may receive signals, in particular data frames, sent by each functional module. This way the communicational module may in a first step identify connected notes of corresponding functional modules.

According to another aspect the electro surgical generator comprises further functional modules, comprising at least one of an energy distribution module for controlling the feed of high frequency energy for one of the inverter modules to one of the output modules. The further functional module may also comprise a display module for displaying information to a user. The further functional module may comprise an input module for receiving an input from a user. All such functional modules have already been explained above. These functional modules also comprise a coupling identifier and can thus be identified after being mounted. Accordingly the step of mounting functional modules also relates to these mentioned functional modules.

This way these additional functional modules may also be identified during manufacturing, i.e. after having been mounted to the casing and/or support frame.

According to one aspect for building up a communication network at least the communication module and/or at least one of the functional modules search for functional modules and autonomously connect to any functional modules found be the search, wherein all functional modules and possibly the communication module connected to each other form the communication network.

Accordingly the communication module searches for functional modules. The communicational module and the functional modules searched for are physically attached to each other in a way that information can be exchanged. However there is no particular protocol for such communication yet. It is also not known which members of such communication network to be built will be there. However ones the communication module finds a functional module they connect to each other.

This means, that a particular protocol and/or provision how to exchange data frames is build up and an identification is also stored in the communication module and/or in the connected functional module. This way these two modules are connected such, that they build a communication network or a part of it. This way it is searched for further functional modules, each physically attached such that they can send information and such further functional modules found are also connected to the communication module and this way a communication network is build up until all functional modules have been found.

However it is also possible that this principle works without the communication module. In this case a decentralized communication network will be build up. Accordingly one functional module searches for a further functional module.

It is also possible, that the communication module and at least one functional module each search for functional modules to build up the communication network. This way it is possible that the communication module and a first of the functional modules have been connected in a first step.

It can also be possible, to set up a wireless communication network but it is preferred to build up a communication network based on communication via wires, as it was found that any electric smog can be problematic due to inverters used in the electro surgical generator which produce high frequency signals that may disturb any communication.

However this aspect for building up a communication network also facilitates the manufacturing of the electro surgical generator in a modular manner. It is thus possible to just mount the functional modules and possibly the communication module, including providing any communicational, i.e. electrical connections for submitting information. In other words a connection using wiring is included when mounting the modules.

It is possible that when mounting the functional modules and/or the communication modules an electrical connection for exchanging information is automatically given when these modules are mounted on a printed circuit board.

According to one aspect based on a given configuration file comprising predetermined configurations of the functional modules, selecting or setting function control modules of functional modules, wherein each function control module is assigned to a functional module to provide a control of that functional module, wherein in particular each function control module is implemented as a software module on the respective functional module, and/or conducting a check, whether the selected and/or set function control modules comply with the predetermined configurations. Accordingly each functional module has a function control module. Such function control module may control the functional module. Controlling the functional module can be performed such that the function control module receives control orders and controls the functional module accordingly. For example if the functional module is an inverter module the function control module of this inverter module may receive an order to start a feed signal. That order may also include the level of the feed signal. Based on this order the function control module initiates the feeding of the feed signal and may control the level of the feed signal as well.

If the functional module is a display module, the function control module may receive information which shall be displayed. The function control module than controls the display module, such that information is displayed.

In order to provide a modular electro surgical generator similar or identical functional modules may be used for different configurations. For example an identical inverter module may be used to provide a feed signal with a first or a second frequency. A first electro surgical generator to be manufactured may need an inverter providing a feed signal with a first frequency whereas a second electro surgical generator may need an inverter providing a feed signal with the second frequency. In this case a physically identical inverter can be used and whether it provides the feed signal with a first or a second frequency is controlled by its control module. The function control module is thus selected or set accordingly.

Which selection or setting to use is given by the configuration file comprising predetermined configurations, including predetermined configuration of the explained function control module so the predetermined configuration specifies whether the first or the second frequency shall be used, to explain it by a way of the above-mentioned example.

To make such selection or setting each function control module may be implemented as a software module of the respective functional module. This way the function control module is already there and its setting can be made based on the predetermined configuration. However it is also possible to provide a plurality of function control modules as a plurality of software modules on the respective functional modules. In that case it is possible to select one of these software modules based on the predetermined configuration. One or a plurality of function control modules may be part of the control base module.

Accordingly the generator can be manufactured by mounting the functional modules. Once the functional modules are mounted and interconnected the particular configuration of the functional modules and thus particular configuration of the generator to be manufactured is still open. However using the given configuration file each configuration and thus each function control module of the functional module can be selected or set. This way many different generators can be manufactured by using the same functional modules. All is needed is to mount the functional modules and then execute the selecting or setting based on the configuration file. Accordingly it is possible to manufacture different electro surgical generators which are only different in their configuration file.

Of course it is possible to nevertheless provide different electro surgical generators which are also different in their physical components, i.e. in their physical functional modules including the number of used functional modules and/or the size of particular functional modules.

According to one suggestion 2, 3 or more electro surgical generators that are different in their size and/or complexity could be manufactured, each forming an electro surgical generator type within each type the components, in particular the functional modules can be physically similar but can be adapted by a configuration file as explained above, such that control modules are selected or set.

It is also possible, that the functional modules and thus the control modules are preset when mounting the functional modules. In that case it is suggested to conduct a check whether the selected and/or set modules comply with the predetermined configurations. Accordingly it is possible to avoid any errors and avoid that a control module is incorrectly selected or set. This way any preselection or presetting may be checked, but it is also possible to double-check the selection and/or setting that has been done based on the predetermined configurations.

According to one aspect the electro surgical generator, in particular the communication device, identifies a connection situation reflecting an overall situation of modules, in particular functional modules, currently installed and/or currently connected in the electro surgical generator, and in particular depending on the identified connection situation it is selected or adapted an overall control function of the electro surgical generator for controlling it.

Accordingly it is suggested to identify an overall situation of modules provided in the electro surgical generator.

Based on that an overall control can be selected or adapted. Such overall control is directed to the interacting of the functional modules. Such interacting may comprise how an inverter module interacts with an output module to which it provides a feed signal.

Set inverter module and also said output module may be connected to a display module in order to display any activity and/or sensor information of the inverter module and the output module. The display may also show a connection situation of the output module, i.e. whether an electro surgical instrument is connected and possibly which kind of instrument is connected.

The setting or selecting of such overall control may include what kind of signals sent by functional modules correspond to what kind of information. For example an inverter module send a value ranging from 0 % to 100 % and the overall control considers which actual physical feed signal corresponds to 100 %. The overall control may also include information on how to communicate with each particular functional module. The overall control may also consider what kind of input signal received from an input module corresponds to what kind of information. For example if there are three buttons at the input module, to give a simple example, each button is related to a particular function. This relationship may for example indicate which button is related to which inverter to be started.

According to the invention there is also suggested a method for manufacturing a plurality of electro surgical generators of different generator composition.

According to this method
- each electro surgical generator is adapted for generating feed signals for feeding electro surgical instruments, and
- each the generator comprises a plurality of functional modules and the plurality of functional modules comprises
   - at least one inverter module for generating a feed signal for providing a high frequency energy for at least one electro surgical instrument,
- at least one output module for connecting an electro surgical instrument to the output module,
wherein
- at least one, a plurality or all of the functional modules comprises a coupling identifier for making the respective functional module distinguishable from other of the functional modules which are different to the respective module, and
for each electro surgical generator the method comprises the steps
- mounting at least the functional modules to a casing or support frame,
- building up a communication between the functional module,
- identifying each functional module by its coupling identifier and
- identifying the generator composition based on the identified functional modules.

Accordingly different electro surgical generators can be manufactured and at the end when all functional modules are mounted and identified the generator composition is identified based on the identified functional modules. As a result the electro surgical generator as such which has just been manufactured can be identified. Such identification can be used to identify the overall generator situation but it can also be used to select or adapt an overall control. This way different electro surgical generators can be manufactured even if physically identical functional modules have been used.

In addition or alternatively each functional module can also be adapted by setting or selecting a control module of the respective functional module, as explained above. This way it is also possible to configure the electro surgical generator, even if physically identical functional modules were used.

According to one aspect it is suggested that each electro surgical generator is manufactured according to a method according to at least one embodiment for manufacturing an electro surgical generator explained above. Accordingly the method as explained above for manufacturing a single generator is used as well but different electro surgical generators are produced by using the same method.

In addition or alternatively it is suggested that at least a first and a second electro surgical generator are manufactured and for the first and second electro surgical generator physically identical functional components are used. Accordingly identical functional components are used but different generators are manufactured. This can be done by adapting the overall control and/or the control of at least one functional module according to specifications given for each of these different electro surgical generators.

According to the invention the generator is thus subdivided in various functional modules which can be understood as hardware modules. On each of this functional modules there is a software implemented. So each functional module may have its own software implemented. The software of these functional modules communicate with each other, in particular using a CAN-bus. This way a generator can be seen as a distributed system, but preferably inside a casing. It is possible that each functional module can provide its objects in a better way, in particular better focus on its function. This way the functional modules are also less dependent on resources of other functional modules or other components.

According to one aspect at least a first and a second electro surgical generator are manufactured each having a similar or identical support frame or similar or identical casing for mounting the functional modules, wherein the first and second electro surgical generators have different functional modules mounted on the similar or identical casing or support frame.

Accordingly this method for manufacturing a plurality of different electro surgical generators provides a modular concept. According to this modular concept there is one base for mounting the components, in particular there is a similar support frame or casing for mounting the functional modules. Accordingly the difference between manufacturing this different generators is only that different functional modules are used. These modules are mounted on the same support frame or casing and accordingly only one type of support frame or casing is needed. The process of mounting the functional components on the support frame or casing is also identical.

A casing may also be synonymously denominated as housing.

However different electro surgical generators can be manufactured using this modular method just by mounting different functional modules. At least one functional module shall be different from another functional module and the same mount seat when compared to the other electro surgical generator manufactured.

A support frame and the casing may also be identical. However there might be small differences such as an additional hole in a front plate of a casing, if the one generator has an additional switch when compared to the other generator to be manufactured. Nevertheless the base i.e. the support frame and/or the casing is similar and the difference between two of such generators manufactured is the functional modules used.

The invention is explained below with reference to the accompanying drawing by way of example of an advantageous embodiment. It shows:
- Fig. 1: a schematic diagram of an electro surgical generator according to an embodiment with an electro surgical instrument connected;
- Fig. 2: block diagram of the electro surgical generator according to Fig. 1;
- Fig. 3: a schematic representation for further functional units using the example of a base module;
- Fig. 4a-c: schematic diagrams showing the arrangement of the modules in different housings; and
- Fig. 5a-c: Perspective illustrations of various embodiment examples for the arrangement of the modules in different housings.

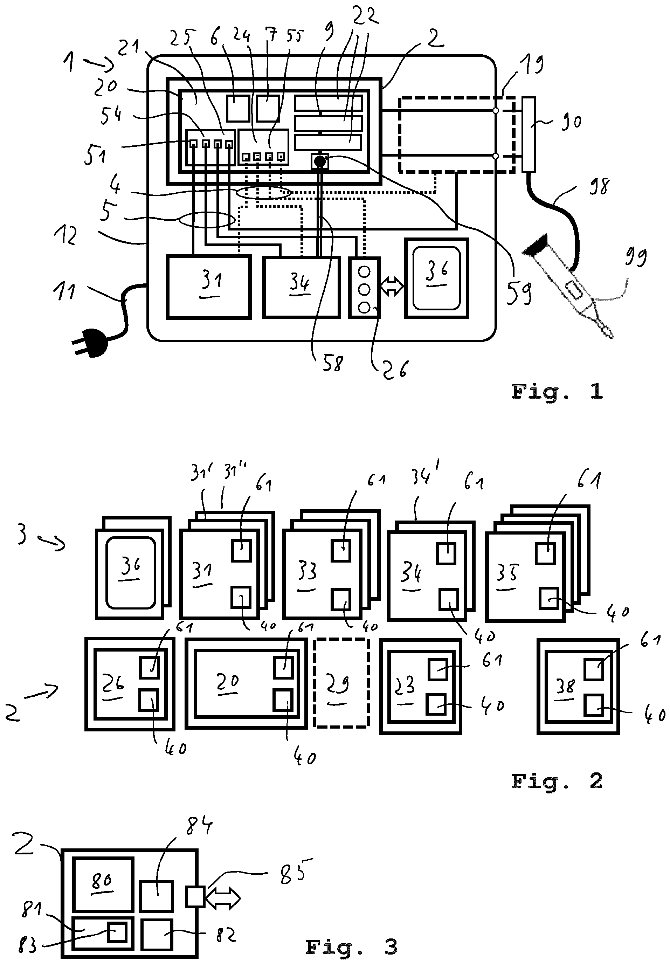

An electro surgical generator 1 according to an embodiment of the invention is shown in Figure 1. The electro surgical generator, designated in its entirety by reference numeral 1, comprises a casing 12 which is provided with an output module 19 which is part of an output module for an electro surgical instrument 99, in the illustrated embodiment example this is an electric scalpel. It is connected to the output port 19 of the electro surgical generator 1 via a connector plug 90 of a high-voltage connection cable 98.

The electro surgical generator 1 is provided with a mains connection cable 11, by means of which it can be connected to and supplied from the public mains supply. It should be noted that other sources may be used for powering, such as a 12 V or 48 V DC power supply for electro surgical generators 1 used in vehicles or other environments with primarily DC power supplies. The power supply cable 11 (or other type of supply, such as the DC power supply) is applied to a power supply module 31, which provides therefrom the voltages required for the operation of the components and modules of the electro surgical generator 1 and distributes them to the individual modules, in particular functional modules and a communication module, via a power supply network 5. Thus, an inverter 34 for generating the output voltage (high voltage) to be delivered to the electro surgical instrument 99 is fed therefrom by the power supply 31.

The inverter module 34 generates a high-frequency AC voltage as an output voltage from the energy supplied by the power module 31. It is typically in the high voltage range, but may have amplitudes in the range of a few 10 V to 4000 V. The type of design of the inverter module 34 for generating the output voltage is open insofar as any of the known concepts, for example single-ended converters or inverters in the narrower sense, can be provided per se. What is essential is that the latter generates the output voltage required for the electro surgical instrument 99 in terms of high frequency and also high voltage. This output voltage thus generated is fed via the distribution module 20 by means of an output voltage line 9 to the output terminal 19 that could also be denominated as an output port, output connection or output socket as synonymous words for connection of the electro surgical instrument 99.

For setting the mode of operation and for displaying certain functions, a display control module 26 is provided, which is shown symbolically in the figure with a user interface having control buttons, via which the user can set the electro surgical generator 1 according to his wishes. It works together with a display, which is designed as a display module 36. The display control module 26 is also representative of an input module.

In this respect, the components to be provided and their functions are basically known. A special feature of the invention lies in particular in the fact that components of the electro surgical generator are of modular design. For this purpose, reference is made to Figure 2, which shows examples of various functional modules from which a selection can be made to create generator types of electro surgical generators 1 which differ in equipment and function. In the bottom line, basic modules 2 are shown, of which at least one must be present and which is typically identical across various electro surgical generators 1 of a series. Basic modules may be functional modules as well.

The basic modules 2 include in particular a distribution module 20, which has a power distribution unit 25 and a communication module 24 and both distributes the electrical power required for operation to the other modules and communicates accordingly with the other modules via the communication module 24 and a communication data network 4 connected thereto. The communication module 24 may be denominated as a communication control module.

It will often be sufficient for a single base module 2 to be provided in the form of the distribution module 20. However, this is not mandatory; it may also be envisaged that particularly complex generators 1, preferably from a different series, are provided with a different base module 2. Further base modules can be in particular the display control module 26 or a rear side module 27 provided for communication or a front side module 23 likewise set up for communication. Optionally, an extension unit 29 can be further provided for the distribution module 20, which is to be coupled only with the distribution module 20, in order to provide so further connection places 22 for additional modules of the electro surgical generator 1. In this way, generator types with very extensive equipment and many individual modules 3 can also be constructed using the same basic module 2.

Shown in the top row of Figure 2 are individual modules which are typically also required for operation of the electro surgical generator 1, but for which there is a choice. In this way, by selecting different individual modules 3, different generator types can be created which differ in terms of equipment and function. In particular, the individual modules include, in addition to the power supply module 31 and the inverter 34, a front panel module 32, at least one connection module 35 for the output connection 19, the display module 36.

The base module 2 and the individual modules 3 are each provided with an interface unit 40. Via this, the respective module is functionally connected to the communication data network 4. The communication data network 4 is designed as a CAN network, divided into two parts with a faster and a slower network.

Reference is now made again to Fig. 1, where a distribution module 20 is shown as base module 2. It comprises a base plate 21 with a power distribution unit 25 and a communication module 24. Furthermore, connectors 54, 55 are provided on the base plate 21 for the connection between the base module 2 and the individual modules 3.

The power supply module 31 is connected via a supply connector 51. The power provided by the power supply module 31 is fed via this to the distribution module 20, which then passes the power on to the base and individual modules 2, 3 via the power distribution unit 25.

In a manner known per se, the inverter module 34 generates an AC voltage from the electrical power supplied by the power supply 31 - via the distribution module 20 - which is to be output as an output voltage to the surgical instrument 99. For this purpose, the AC voltage output by the inverter 34, which is typically a high voltage, is conducted via a high voltage line connection 58 to the distribution module 20, specifically to a high voltage connection 59 arranged there. From the high voltage connection 59, the high voltage is applied via an output voltage line 9 integrated in the distribution module 20. The output voltage line 9 is routed, inter alia, to a plurality of receiving locations 22 on the base plate 21, wherein a connection module 35 for the electro surgical instrument 99 can be received in each of the receiving locations 22.

In this way, at least one and optionally several connection modules 35 can be supplied with the high voltage generated by the inverter 34, namely simply by inserting these connection modules 35 into their respective receptacle 22. If, for example, an electro surgical generator 1 of a different generator type has additional output connections 19', then only additional connection modules 35 are to be provided for this purpose, which are simply to be inserted in each case into one of the receptacle locations 22 and are then automatically supplied, namely also with the high voltage generated by the inverter 34. In this way, even complex electro surgical generators 1 with a plurality of different connections 19 can be realized in a simple modular manner, namely by simply inserting corresponding connection modules 35 into one or more of the provided receptacles 22.

It is understood that the pickup stations 22 are also connected to the power distribution unit 25 and communication module 24 to be appropriately powered (not shown in Figure 1).

Thus, a triple supply, namely for the regular power supply, the data communication connection as well as for the high voltage as output voltage, is formed with respect to different types of individual modules 3 to be connected including the connection modules 35. In the described embodiment example, the latter are to be inserted directly into the distribution module 20 and are then directly connected to the output voltage conducted as high voltage in the output voltage line 9 as well as connected to the power distribution unit 25 and communication module 24 which can also be denominated as a communication module. The remaining individual modules 3 are typically connected to the power distribution unit 25 and communication module 24 via cable connections. For this purpose, they are connected to the communication module 24 via connector plugs 54 and to the power distribution unit 25 of the distribution module 20 via connector plugs 55, respectively. This ensures a high degree of flexibility, particularly with regard to the spatial arrangement of the individual modules 3, as is advantageous, among other things, with regard to housings 12 of various sizes and the resulting varying installation position or spacing.

The electro surgical generator 1 also has a module detector 6. This is designed to determine the modules mounted and connected in the electro surgical generator 1 and their configuration. The module detectors can be arranged decentral on the respective modules 2, 3. In the embodiment example shown, however, the module detector 6 is arranged centrally, namely on the distribution module 20 of the base module 2. The module detector 6 is connected to the power supply network 5 and the communication network 4 via lines (not shown in Fig. 1). The module detector 6 is automated. Via the communication network 4, the module detector 6 communicates with the various modules 2, 3 of the electro surgical generator 1. In doing so, the module detectors determines which modules 2, 3 are mounted and active. The module detector 6 thus determines which modules 2, 3 are actually present and how these modules 2, 3 are configured. In this way, the module detector 6 detects the module equipment of the electro surgical generator 1. This can be used, for example, to detect the actual equipment of the electro surgical generator 1.

The module detector 6 further interacts with a self-configuration unit 61 on the modules 2, 3. For this purpose, the modules 2, 3 each have a self-configuration unit 61. By means of the self-configuration unit 61, the respective module 2, 3 can configure itself automatically, depending on the respective determined actual equipment, i.e. the real existing module equipment of the electro surgical generator 1. For example, the inverter module 34 which generates the high voltage for the electro surgical instrument 99 can be configured depending on the type of output module 19 and its output module 35. Thus, the output voltage or the mode of delivery of the output voltage, in particular its waveform, consequently the "modes" of the inverter module 34 can be automatically configured depending on the output port 19 to be supplied and its connection module 35. The module for the power supply 31 feeding the inverter module 34 is then in turn configured by its own self-configuration unit 61 with respect to the voltage or power to be delivered depending on the configuration of the inverter module 34 set - as described above. In this way, a practically complete self-configuration of the modules 2, 3 of the electro surgical generator 1 can be achieved.

Thanks to the decentral arranged self-configuration unit 61, this is also possible when modules 2, 3 are replaced or a new individual module 3 is added.

Furthermore, an enabling unit 7 is provided. In the embodiment shown, it is arranged centrally, namely on the distribution module 20. The enabling unit 7 is designed to determine enabled functions depending on the actual module configuration (e.g. determined by the module detector 6) and to transmit corresponding enabling signals to the modules 2, 3. In this way, the functionality of the individual modules 2, 3 can be enabled or disabled as a whole, and/or the scope of their respective functionality can be determined and set, depending on the actually existing module configuration. For this purpose, the enabling unit 7 transmits corresponding enabling signals to the modules 2, 3 via the communication network 4. This ensures that only those modules 2, 3 or their functionality are enabled and activated which match the respective electro surgical generator 1 and the configuration with the modules 2, 3 installed there.

Reference is now made to Figure 3, which schematically shows further functional units that can be arranged on the modules 2, 3. This is explained by way of example in Figure 3 for the base module 2; this applies accordingly to the individual modules 3 and thus for any functional modules as well. The base module 2 further comprises a processor unit 80, which is provided with its own control program. This is stored in a memory 81. Thus the base module 2 has an independent local operating control system which, like the base module 2, is connected to the generator-internal power supply network 5 and communications data network 4. This applies accordingly to the individual modules 3 equipped in the same way. With their own processor unit 80 and the stored control program, modules 2, 3 are self-sufficient in this respect and do not require any external control (whereby the term "external" refers to the respective module, in the sense of "module-external").

Further functional units can also be provided, which can be arranged locally on one (central) or more of the modules 2, 3 (decentralized). In Figure 3, this is shown by way of example using the basic module 2; the same can apply to the individual modules 3. Accordingly, a safety unit 82, that can also be denominated a backup unit, is further provided with regard to the module configuration. The backup unit 82 is designed to compare a real module configuration, as determined for example by the module detector 6, with a target equipment characteristic 83. The target equipment characteristic 83 is a data record for the configuration of the modules 2, 3 as intended by the manufacturer. During commissioning or by the user in the course of normal operation, the module configuration during normal operation, the safety unit 82 compares the actual module configuration, as determined in particular by the module detector 6, with the stored nominal equipment characteristic 83. If there is agreement, the configuration of the electro surgical generator 1 is OK and operation can take place as intended. However, if a discrepancy results, there is either a defect in one of the modules 2, 3 or a misconfiguration of at least one of the modules 2, 3. A corresponding error signal is output, preferably via the data communication network 24 and indicated to the user on the display 36, and subsequently the operation of the electro surgical generator 1 is blocked for safety. This blocking can be complete or partial, depending on the discrepancy, so that, if necessary, certain functions are blocked and the electro surgical generator 1 can continue to be operated with the unaffected functions or its basic functions. All this is done automatically by the backup unit 82.

Furthermore, an upgrade unit 84 can be provided, which interacts with a secure communication link 85. Via this, the upgrade unit can communicate with the outside world and, in particular, can be controlled by an external authorized body (for example, the manufacturer). The upgrade unit acts in conjunction with the secure unit 82 and the memory 81 to provide the target equipment feature 83 with an authorized change, if necessary. In this way, additional functions can be enabled or further additional modules 3, which have been subsequently installed, can be authorized for operation of the electro surgical generator 1 and integrated into it.

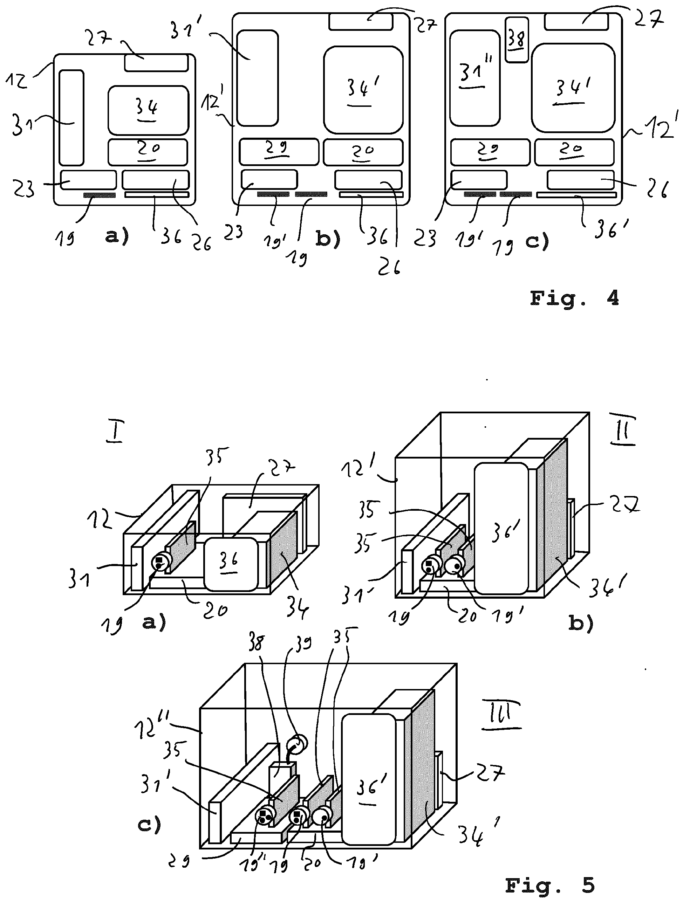

Figures 4a-c show different types of generators with their respective function blocks. The generator type shown in Figure 4a is a basic version. It comprises as basic modules 2 a distribution module 20, a display control module 26 and a front side module 23. Furthermore, as individual modules 3 a module for power supply 31, a module for inverter 34, a rear side module 27 and a display module 36, which is arranged in the front panel, are provided. Also arranged on the front panel is the output connector 19 for the electro surgical instrument 99 (see Fig. 1). Figure 4b shows another type of generator, which differs from those shown in Figure 4a essentially in having a stronger power supply module 31', a larger and more powerful module for the inverter module 34', and a second output port 19'. For the two output ports 19, 19', in order to accommodate their respective output modules 35 (not shown in Figure 4), the distribution module 20 is supplemented by an extension unit 29 so as to provide at least one additional accommodation space 22 (not shown in Figure 4). In order to provide sufficient space for this, a larger casing 12' is provided. It can be seen that a free space remains in the center which, if necessary, can be used for further equipment with individual modules 3 or an optional module 14 (each not shown in Figure 4b) thanks to the modular concept. Figure 4c again shows another type of generator. This differs from the one shown in Figure 4b essentially in that an additional individual module 3 in the form of an ultrasonic module 38 is provided. In order to supply this also sufficiently with power, another module is provided for the power supply, namely an amplified power supply 31".

The individual modules mentioned in all figure are representative for functional modules.

Figure 5a-c shows three different series of generator types I, II and III in perspective view. They serve to illustrate the arrangement of the modules in different housings. The first series I shown in Fig. 5a is a basic design. A module for the power supply 31 on one side, an inverter module 34 on the other side as individual modules 3 and in between the distribution module 20 as basic module 2 are arranged in a rather smaller casing 12. A modular front panel with a display module 36 is arranged on the front side. A display control module 26 is arranged on the rear side of the display module 36 (not visible in Fig. 5, cf. Figs. 4a-c). A rear module 27 for connecting external components is provided on a rear wall of the casing 12. Plugged into the distribution module 20 is a connection module 35 which outputs the output voltage generated by the inverter via an output connection 19.

Fig. 5b shows a middle series II. Compared to series I, it features a different individual module for the inverter, namely one with a more powerful inverter 34'. Further, a larger display module 36' is provided, which is controlled by the display drive module 26. All of this necessitates a larger casing 12'. A further connection module 35 is also plugged into the base module 2, which outputs output voltage via a further output connection 19'.

In Fig. 5c, a higher-order series III is shown, which is similar to series II but has a further output connection 19". For this purpose, a further connection module 35 is provided, which is plugged into an extension unit 29 for the base module 2. Furthermore, an ultrasonic module 38 is provided as an optional module 14, which is arranged in the free space between the module for power supply 31' and the inverter module 34'. It is connected to the communication network 4 and the power supply network 5 via cable connections (not shown). All this necessitates a large casing 12". An additional RF output port 39 is provided at the rear thereof to which the ultrasonic module 38 is connected for output of ultrasonic energy.

As can be seen from these examples, a large number of different generator types and series can be generated in this way with just a few modules 2, 3 and minor modifications.

Further aspects are defined by the following embodiments which can be combined with any of the aspect explained above and defined by the claims.

Embodiment 1: Electro surgical generator for generating feed signals for feeding electro surgical instruments, and the generator comprises a plurality of functional modules and the plurality of functional modules comprises
- at least one inverter module for generating a feed signal for providing a high frequency energy for at least one electro surgical instrument, and

- at least one output module for connecting an electro surgical instrument to the output module,
wherein
- at least one, a plurality or all of the functional modules comprises a coupling identifier for making the respective functional module distinguishable from other of the functional modules which are different to the respective module,
- at least one of the functional modules or a communication module of the electro surgical generator are adapted to identify the respective functional module by the coupling identifier, and wherein
- a bus system is provided for communication between the functional modules and/or with the communication module, and/or
- each of a plurality or all of the functional modules have a control base module for controlling the respective functional module and/or for operating a communication of the respective functional module with another functional module and/or with the communication module,
characterized in that for communication
- the communication module is interconnected with the functional modules via communication lines, and/or
- the functional modules are interconnected with each other via communication lines and/or
- a bus system is used, in particular a bus system using a serial and/or differential data transfer, in particular a CAN-bus.

Embodiment 2: Electro surgical generator according to embodiment 1, characterized in that
- the electro surgical generator, in particular the communication module,
- uses a plurality of bus systems, having different transfer rates.

Embodiment 3: Electro surgical generator according to embodiment 1 or 2, characterized in that
- a plurality or all of the functional modules each comprise a data interface for building up a communication or for participating in a communication with the communication module and/or with other functional modules.

Embodiment 4: Electro surgical generator according to any of the preceding embodiments, characterized in that
- at least one of the functional modules comprises settable or selectable module configurations, and in particular
   - the electro surgical generator is adapted that for each functional modules a module configuration is set or selected in dependence of an identified set up of the electro surgical generator, and in particular
   - each functional module having settable or selectable module configurations comprises a configuration unit, and/or
- a central configuration unit is provided, in particular as part of the communication module.

Embodiment 5: Electro surgical generator according to any of the preceding Embodiments, characterized in that
- an enabling function is implemented wherein the enabling function is adapted to enable a particular functional module to operate and/or to execute a particular function, such that the particular functional module can only operate or execute the particular function if it is enabled by the enabling function, wherein
- for enabling
   - a central enabling unit is provided, and/or
   - decentralized enabling units are provided such that each functional module comprises an enabling unit.

Embodiment 6: Electro surgical generator according to any of the preceding embodiments, characterized in that
- the electro surgical generator comprises a configuration file, having stored an intended setup of the electro surgical generator, defining which functional modules to be used in the electro surgical generator, and/or
- the electro surgical generator is adapted to compare, in particular by means of a comparator, the setup of the electro surgical generator stored in the configuration file with an actually identified set up of the electro surgical generator.

Embodiment 7: Electro surgical generator according to any of the preceding embodiments, characterized in that it further comprises
- a display module,
- a display control module having a control algorithm for controlling a display module, in particular being adapted to identify a display module installed in the electro surgical generator and adapt the control algorithm to the identified display module,
- an external communication module having a communication protocol for communicating with external applications connected to the external communication module, in particular being adapted to identify connected external applications, and to adapt the communication protocol to the identified external applications,
- an input module for enabling a user to give inputs to the electro surgical generator, and
- a module detector for detecting functional modules installed in the electro surgical generator.

Embodiment 8: Electro surgical generator according to any of the preceding embodiments, characterized in that
- the electro surgical generator is adapted to receive and process an upgrade signal, in particular the electro surgical generator comprises an upgrade processing unit for receiving and processing an upgrade signal.

Embodiment 9: Electro surgical generator according to any of the preceding embodiments, characterized in that
- the electro surgical generator comprises a support frame or casing on which all functional modules and in particular also all other modules are mounted, and in particular
- the support frame or casing comprises a power supply for supplying electrical power to the electro surgical generator, in particular to all modules that need electrical power.

Embodiment 10: Method for manufacturing a plurality of electro surgical generators of different generator compositions, wherein
- each electro surgical generator is adapted for generating feed signals for feeding electro surgical instruments, and
- each generator comprises a plurality of functional modules and the plurality of functional modules comprises
   - at least one inverter module for generating a feed signal for providing a high frequency energy for at least one electro surgical instrument,
- at least one output module for connecting an electro surgical instrument to the output module,
wherein
- at least one, a plurality or all of the functional modules comprises a coupling identifier for making the respective functional module distinguishable from other of the functional modules which are different to the respective module, and
for each electro surgical generator the method comprises the steps
- mounting at least the functional modules to a casing or support frame,
- building up a communication between the functional module,
- identifying each functional module by its coupling identifier and
- identifying the generator composition based on the identified functional modules.

Embodiment 11: Method according to embodiment 10, characterized in that
- each electro surgical generator is manufactured according to a method of any of claims 11 to 15, and/or
- at least a first and a second electro surgical generator are manufactured, and
for the first and second electro surgical generator physically identical functional components are used.

Embodiment 12: Method according to embodiment 10 or 11, characterized in that
- at least a first and a second electro surgical generator are manufactured, each having a similar or identical support frame or similar and/or identical casing for mounting the functional modules, wherein
- the first and second electro surgical generators have different functional modules mounted on the similar or identical casing or support frame.

## Claims

1. Electro surgical generator for generating feed signals for feeding electro surgical instruments, and the generator comprises a plurality of functional modules and the plurality of functional modules comprises
- at least one inverter module for generating a feed signal for providing a high frequency energy for at least one electro surgical instrument, and
- at least one output module for connecting an electro surgical instrument to the output module,
wherein
- at least one, a plurality or all of the functional modules comprises a coupling identifier for making the respective functional module distinguishable from other of the functional modules which are different to the respective module,
- at least one of the functional modules or a communication module of the electro surgical generator are adapted to identify the respective functional module by the coupling identifier, and wherein
- a bus system is provided for communication between the functional modules and/or with the communication module, and/or
- each of a plurality or all of the functional modules have a control base module for controlling the respective functional module and/or for operating a communication of the respective functional module with another functional module and/or with the communication module.

2. Electro surgical generator according to claim 1, **characterized in that** the plurality of functional modules further comprises
- an input power module for supplying the electro surgical generator with electrical power, and/or
- an energy distribution module for controlling the feed of power from the or at least one of the inverter modules to the or at least one of the output modules, and/or
the electro surgical generator further comprises:
- the communication module coupled with two or more of the functional modules for providing a communication between the communication module and/or the two or more functional modules and/or
- a module detector for detecting which functional modules are mounted and/or are configured.

3. Electro surgical generator according to claim 1, **characterized in that**
- two or more of the modules each having a communication interface for communicating with other modules and/or with the communication module and/or
- a self-configuring communication network is provided, and in particular
- at least the communication module and/or at least one of the functional modules are adapted to search for communication members connected to the network and to autonomously connect to any members found by the search.

4. Electro surgical generator according to claim 1 or 2, **characterized in that**
- a plurality of the functional modules each have a control base module for controlling the module, wherein
- each of these functional modules is prepared for providing at least one module function, and
- in the control base module a modular control method is implemented, that has at least
- a communication control module for operating the communication of the module and
- a function control module for controlling the function of the module.

5. Electro surgical generator according to any of the preceding claims, **characterized in that**
- in each of at least one of the plurality of functional modules,
- a plurality of function control modules is provided, each as a software control unit for controlling different module functions of a functional module such that each module function is assigned to a function control module and
- for selecting a module function a corresponding function control module is selected.

6. Electro surgical generator according to any of the preceding claims, **characterized in that**
- for controlling each module
- a plurality of control base modules are provided as software units and
- for varying of a functionality of the functional module at least one of the control base modules is changeable and/or it can be chosen between some of the control base modules, wherein in particular
- at least one of the control base modules is provided as fixed, unchangeable control base module.

7. Electro surgical generator according to any of the preceding claims, **characterized in that**
- a configuration file is given, including predetermined configurations in order to select and/or set control base modules, which are particularly given as software units, and optionally
- the electro surgical generator, in particular the communication device, is adapted to conduct a check, whether the actually chosen and/or set control base module matches the predetermined configuration.

8. Electro surgical generator according to any of the preceding claims, **characterized in that**
the electro surgical generator, in particular its communication device, is adapted to execute, one, a plurality or all of the communication steps of the list comprising:
- receive information from functional modules,
- evaluate the information received,
- extract control commands from the evaluated information,
- transmit control commands extracted from the evaluated information,
- transmit received information to other functional modules and
- check if transmitted information, in particular transmitted control commands, matches the functional module to which they were transmitted.

9. Electro surgical generator according to any of the preceding claims, **characterized in that** the communication device is
- coupled to the modules via communication wires, and/or
- connected to the bus system for communication, in particular to a serial and/or differential bus system, and/or **characterized in that**
- the electro surgical generator, in particular the communication device,
- comprises or uses a plurality of bus systems having different rates of transmission, and/or
- the electro surgical generator, in particular the communication device, is adapted to select a transmission rate for exchanging information,
- depending on information to be transmitted and/or depending on the module to be controlled.

10. Electro surgical generator according to any of the preceding claims, **characterized in that**
- the electro surgical generator, in particular the communication device, is adapted to
- identify a connection situation reflecting an overall situation of modules currently installed and/or currently connected in the electro surgical generator, and in particular
- depending on the identified connection situation, select or adapt an overall control function of the electro surgical generator for controlling the electro surgical generator.

11. Method for manufacturing an electro surgical generator, which is adapted for generating feed signals for feeding electro surgical instruments, and the generator comprises a plurality of functional modules and the plurality of functional modules comprises
- at least one inverter module for generating a feed signal for providing a high frequency energy for at least one electro surgical instrument,
- at least one output module for connecting an electro surgical instrument to the output module,
wherein
- at least one, a plurality or all of the functional modules comprises a coupling identifier for making the respective functional module distinguishable from other of the functional modules which are different to the respective module, and
the method comprises the steps
- mounting at least the functional modules to a casing or support frame,
- building up a communication between the functional modules, and
- identifying each functional module by its coupling identifier, and
- using a bus system for communication between the functional modules and/or with the communication module and/or
wherein
- each of a plurality or all of the functional modules have a control base module for controlling the respective functional module and/or for operating a communication of the respective functional module with another functional module and/or with the communication module and
wherein in particular
- an electro surgical generator according to any of the preceding claims is manufactured.

12. Method according to claim 11, **characterized in that**
- based on the identified functional modules a generator composition is identified, and/or
- the electro surgical generator further comprises
- a communication module for controlling the communication between the functional modules and/or for controlling the building up of the communication,
- further functional modules, comprising at least one of
- an energy distribution module, for controlling the feed of high frequency energy from one of the inverter modules to one of the output modules,
- a display module for displaying information to a user, and
- an input module for receiving an input from a user.

13. Method according to claims 11 or 12, **characterized in that**
- for building up a communication network,
- at least the communication module and/or at least one of the functional modules search for functional modules and autonomously connect to any functional modules found by the search, wherein
- all functional modules and possibly the communication module connected to each other form the communication network.

14. Method according to any of claims 11 to 13, **characterized in that**
- based on a given configuration file comprising predetermined configurations of the functional modules,
- selecting or setting function control modules of functional modules, wherein each function control module is assigned to a functional module to provide a control of that functional module,
- wherein in particular each function control module is implemented as a software module on the respective functional module, and/or
- conducting a check, whether the selected and/or set function control modules comply with the predetermined configurations.

15. Method according to any of claims 11 to 14, **characterized in that**
- the electro surgical generator, in particular the communication device,
- identifies a connection situation reflecting an overall situation of modules, in particular functional modules, currently installed and/or currently connected in the electro surgical generator, and in particular
- depending on the identified connection situation, selects or adapts an overall control function of the electro surgical generator for controlling it.
